# EUROPEAN PATENT APPLICATION

(11) **EP 1 783 646 A1**
(43) Date of publication of application: **09.05.2007**
(21) Application number: 06022934.1
(22) Date of filing: 03.11.2006
(51) Int. Cl.: G06F 19/00

(54) **System for detection and correction of errors in input data for genotype analysis**

(30) Priority: 08.11.2005 JP 2005323401
(71) Applicant: HITACHI SOFTWARE ENGINEERING CO., LTD., Yokohama-shi, Kanagawa 230-0045 (JP)
(72) Inventor: Matsumoto, Toshiko, Tokyo 140-0002 (JP); Nakashige, Ryo, Tokyo 140-0002 (JP)
(74) Representative: Liesegang, Roland

(57) **Abstract**

A data input support system is provided to preliminarily remove particular error causes when genotype data are input for a program to execute linkage disequilibrium analysis or the like. By taking advantage of limiting conditions characteristic of genotype input data and the statistical properties of the entire data set, possible errors are detected by a preprocessing program, the detected errors are associated with false descriptions causing them to report the results, user input responding to the reported results is accepted, and a modified version of the input data is output.

## Description

### BACKGROUND OF THE INVENTION

### Field of the Invention

The present invention relates to a support system for inputting appropriate genotype data into an analysis system in gene analysis to identify genes associated with phenotypes of diseases, physical appearance features or the likes in individuals.

### Background Art

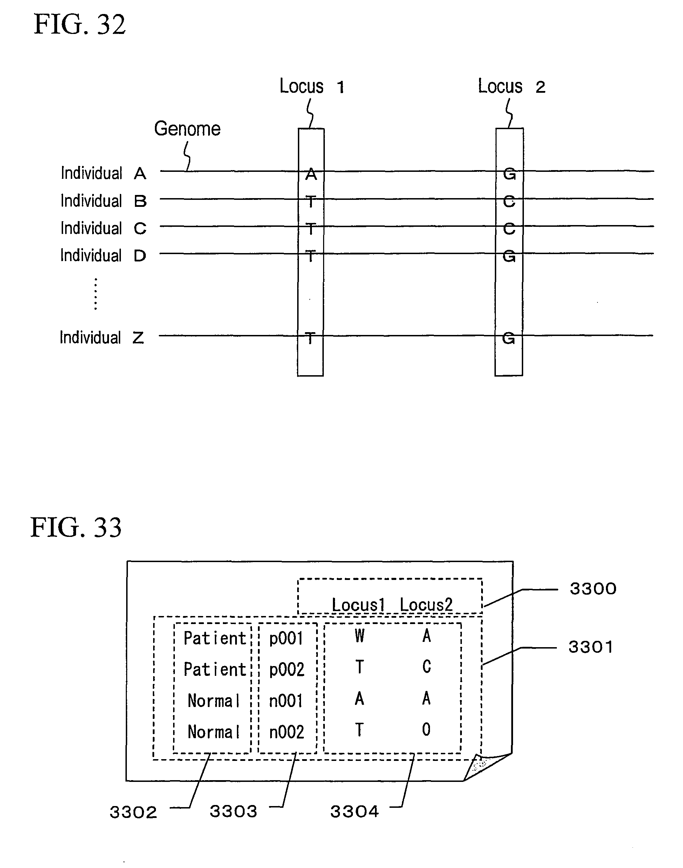

Genome mapping has been advanced for the human, animals and plants and analytical studies on gene functions are actively under progress. Of those studies, attract a particular attention studies through analysis of linkage disequilibrium which are to search the genome for genes associated with phenotypes (traits) of diseases, physical appearance features or the likes in individuals. As shown in FIG. 32, a case will be now discussed where individuals A to Z of the same species are compared with respect to genome. Individuals of the same species have generally very similar nucleotide sequences, but different nucleotides in some positions. In FIG. 32, the individuals have different nucleotides in loci 1 and 2. Here, the term "locus" refers to a specific location in a genomic nucleotide sequence.

Such a polymorphic occurrence of a single nucleotide in the genome among individuals is called SNP (single nucleotide polymorphism). A single locus is typically occupied by either of two different nucleotides (for example, A and T), but may be occupied by any one of three or more different nucleotides (for example, A, T and G) in very rare cases. In the case shown in FIG. 32, more individuals have T in locus 1, and therefore T is termed "major" in locus 1, while A is termed "minor." Similarly, G is termed "major" in locus 2, while C is termed "minor."

A case where the same locus is occupied by A in an individual but by no nucleotide in another individual, or a similar case may also happen. In this case, if the first individual views the genome of the latter individual, it is observed to have deletion of a nucleotide A, but if the latter individual views the genome of the first individual, conversely, it is observed to have insertion of the nucleotide A. Such a polymorphic presence/absence of a single nucleotide in the same locus among individuals is called in/del (abbreviation of insertion/deletion) of the single nucleotide.

On the other hand, individuals of many biological species have a pair of genomes (homologous chromosomes) derived from both a female gamete and a male gamete. Genes present at sites corresponding to one another in the pair of genomes are called alleles to one another, and a pair of these alleles is called a genotype. The two alleles may be the same or different since there are different nucleotide sequence portions among individuals in genome. When genes at a particular genomic site are paid attention to, the presence of the same two alleles is called homozygotes, while the presence of different two alleles is called heterozygotes.

When chromosomes are transferred from a parent to a child, the single genome undergoes crossing-over by meiosis and thus gene recombination in the transfer. It is generally believed that two distant genes in the genome are likely to be recombined, but two near genes in the genome are difficult to be recombined. When genes located at two different loci in the genome tend to be transferred from a parent to a child as they are linked, the expression that the two loci have a linkage is used.

Genetic search of hereditary diseases associated with a small number of genes has been conducted up to now by linkage analysis using a program such as "LINKAGE" where data of a large family including at least one patient are input.

### An example of linkage analysis programs: LINKAGE

It was developed by Rockefeller University in USA. Genotype data of a large family including at least one patient are used for linkage analysis. ftp://linkage.rockefeller.edu/software/linkage/

On another front, in the search of genes which affect multifactorial diseases attracting current attention (diseases such as lifestyle-related diseases which afflict numerous patients and are probably associated with many genes as well as environmental factors), analysis of linkage disequilibrium is actively conducted for which a general population without blood relationship is used, as described below.

In a single genome derived from either a female gamete or a male gamete, a set of alleles present at multiple linked loci is called a haplotype. Individuals having two homologous genomes in a pair have always two haplotypes in a pair:

A phenomenon may be occasionally observed where the frequency of a certain haplotype for multiple linked loci is significantly different from a frequency which is given by product of frequencies for alleles at the respective loci (the alleles are distributed interdependently among the multiple linked loci). In this case, the expression that those loci are at linkage disequilibrium is used.

The above analysis of linkage disequilibrium can be used to search the genomes of individuals for genes associated with phenotypes (traits) of diseases, physical appearance features or the likes. Two approaches to the analysis will be described below. The first approach will be now described. It is assumed that most of genes responsible to common diseases in a population are formed by mutation of common ancestor genes (common disease common variant assumption). According to the assumption, an SNP allele close to the locus where such mutation occurred would be inherited in a combination with the pathogenic gene. In other words, linkage disequilibrium would be observed between the locus for the pathogenic gene and SNP loci close thereto. Therefore, such a region in the genome is called a linkage disequilibrium block or a haplotype bloc. A haplotype block common to individuals suffering from a certain disease can be searched to identify a gene causing the disease. The second approach will be now described. If the SNP allele close to the mutated gene is inherited to the patient population together with the pathogenic gene, as described above according to the common disease common variant assumption, the frequency of the allele would be different between the patient population and the healthy population. This deduction draws the assumption that conversely, an SNP allele having a different frequency between the patient population and the healthy population would be accompanied by a pathogenic gene close thereto. An approach of combining multiple SNPs to form a haplotype is similarly used to compare its frequency between the patient population and the healthy population.

When genes associated with phenotypes are searched for using linkage disequilibrium analysis, tens to hundreds of individual samples, sometimes at least a thousand of those, are typically used to examine genotypes at several to hundreds of loci, sometimes about ten thousand loci. In addition, many programs for linkage disequilibrium analysis using genotypes as input data have been developed and are now available as described below.

### Example 1 of programs for linkage disequilibrium analysis: ARLEOUIN

It was developed by University of Geneva in Switzerland. Genotypic data of unrelated individuals are used to test the Hardy-Weinberg equilibrium and calculate for linkage disequilibrium.
Stefan Schneider, David Roessli, and Laurent Excoffier (2000) Arlequin ver. 2000: A software for population genetics data analysis. Genetics and Biometry Laboratory, University of Geneva, Switzerland.

### Example 2 of programs for linkage disequilibrium analysis: Haploview

It was developed by Whitehead Institute in USA. Genotypic data of unrelated individuals are used to verify the number of missing samples for each locus, verify the Hardy-Weinberg equilibrium (described later), verify distances among loci, verify the frequencies of minor alleles and calculate for haplotype blocs (see J. C. Barrett, B. Fry, J. Maller and M. J. Daly, "Haploview: analysis and visualization of LD and haplotype maps", Bioinformatics vol.21, no.2 (2005), pages 263-265).

### Example 3 of programs for linkage disequilibrium analysis: Varia

It was developed by Silicon Genetics Inc. in USA (as of filing the present patent application, the same software known as "GeneSpring GT" is available from Agilent Technologies Inc. in USA). Genotypic data of family or unrelated individuals are used to carry out data analyses such as calculation for haplotype blocs. http://www.silicongenetics.com/cgi/SiG.cgi/Products/Varia/features. smf

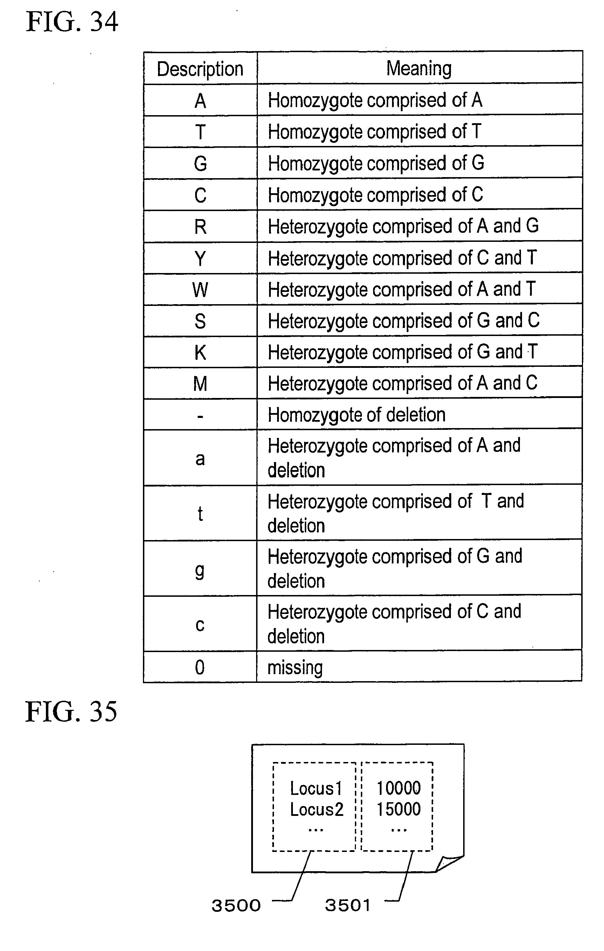

IUB code, which is described in Fig. 33, is one of description formats for input data (genotype data) which is used in a program to carry out linkage disequilibrium analysis. In the IUB coding system, names of loci are described one after another on the first line (3300), and the data of respective individuals are described on the second and following lines (3301). In the description of the respective individual data, the presence/absence of a disease is described at the leftmost place on the line (3302), an individual identifier is described next (3303), and then genotypes carried by the individual are described one after another according to the order of the loci described on the first line (3304). As for the presence/absence of a disease, patients are described as "Patient", while healthy individuals are described as "Normal". Genotypes are described by IUB codes shown in FIG. 34. In the example shown in FIG. 33, the individual p001 is a patient, is a heterozygote comprised of A and T at locus 1, and is a homozygote comprised of A at locus 2. The term "missing" means no genotype data available due to experimental failure or the like. Of the genotypic descriptors shown in FIG. 34, "-", "a", "t", "g" and "c" are used in in/del polymorphism.

In addition, algorithms taking account of distances between loci (by how many nucleotides are the two loci separated?) for calculations have been proposed to determine haplotype blocs. Therefore, location of each locus is necessary to be specified in its input data. Fig. 35 is one of formats for such location. In this format, the data of each locus is described on each line, where the name of each locus is described in the leftmost place of each line (3500) and its physical position (of what number is the nucleotide in order starting from the top of the chromosome?) is described next (3501).

When a pathogenic gene is searched for with the help of a program, it is problematic that false descriptions are present in the input data. The program assumes that the input data given is perfectly correct. However, genotype data obtained experimentally are often processed into electronic data or changed in format in manually, and hence it is almost impossible to completely prevent false descriptions in the input data. In addition to errors made in manual input of the data, errors may be brought in from wrong experimental results. Taking them together, numerous errors may happen.

For conventional linkage analyses, the approach of making sure if genotype data are consistent or not by use of parenthood is presented such as Varia or Checkfam.

### Example of contradiction detection programs for genotype data in linkage analysis: Checkfam

It was developed by Tokyo Women's Medical University. Genotypic data with information of families are used to search them for contradiction as to inheritance of alleles. http://www.genstat.net/checkfam/index.cgi?lang=ja

As for input data for linkage disequilibrium analysis, however, no correction measures have ever been taken though various errors may occur as described below.

### Error 1: no data of physical positions of loci are provided in the input data for a program requiring them

In this case, input files are not so adequate as to execute the analysis program.

### Error 2: loci are not arranged in order of their physical positions (in a chromosome) in the input data for a program where the loci are assumed arranged correctly

In this case, the program may abnormally terminate on the way, or analysis results may be different from those intended even if the program can be executed. When the program has been apparently executed to the end, there is a risk that the researcher may not recognize that analysis results are different from those intended.

### Error 3: some loci are present in the same physical position in the input data for a program where physical positions of loci are not assumed overlapped

There is a risk that physical positions of loci may become inconsistent and overlapped depending on how they are re-counted when the genomic sequence data of the chromosome is updated, or how they are counted for in/del polymorphism.

### Error 4: no genotype data is specified for a particular locus/the physical position is not specified

Some SNPs have multiple locus names due to the process of their discovery. In addition, in the description of locus names, "(ABI)" may be appended to the locus names of SNPs developed by Applied Biosystems Inc., and "(JSNP)" may be appended to the locus names of SNPs developed by the JSNP project. In this case, there is a risk that the additional character strings may drop off or turn into double-byte characters while the input data are produced manually. When inconsistent locus names are produced by these causes, a particular locus is processed by a program as if no genotype data therefor were specified/the physical position thereof were not specified. Such a situation is time-consuming to find out a cause for the problem and solve it.

### Error 5: unexpected character strings are used to represent genotypes

In the IUB codes shown in FIG. 34, "0" is intended to denote missing data. However, a symbol such as "*" (asterisk) or the like may be used by mistake to denote missing data. Or, "AT", the continuous form of the two alleles, rather than "W" may be used by mistake again to denote a heterozygote comprised of A and T. In this situation, the program may abnormally terminate on the way due to appearance of the unexpected character string.

### Error 6: individuals belonging to an unexpected population are used/only one of the populations is provided in the input data for a program where a patient population and a healthy population are intended for analysis

In the format shown in FIG. 33, it is intended that a patient is described as "Patient" and a healthy person as "Normal". By mistake, however, the patient may be described as "Case", or the healthy person may be described as "Control". In addition, a capital letter may be accidentally replaced by a lower-case letter. Furthermore, a something beginning with "P" should be specified as an identifier for the patient and a something beginning with "N" as an identifier for the healthy person, but in some cases, the presence/absence of a disease may be omitted. In these situations, the program may abnormally terminate on the way.

### Error 7: a locus comprising three or more alleles is present

Four causes can be presumed as follows.

The first cause is that three or more alleles have been actually present at the locus, and thus it is not a false description. However, the feature of an experimental technique taken must be considered because the base sequence reading experiment or the use of a DNA microarray allows three or more alleles to be differentiated, but the TaqMan assay or the like may allow only two alleles to be differentiated. Some programs directed to SNP are based on the assumption that each locus has two alleles. In such programs, a relevant locus must be removed from the analysis, or the least frequent allele must be combined with the most frequent allele.

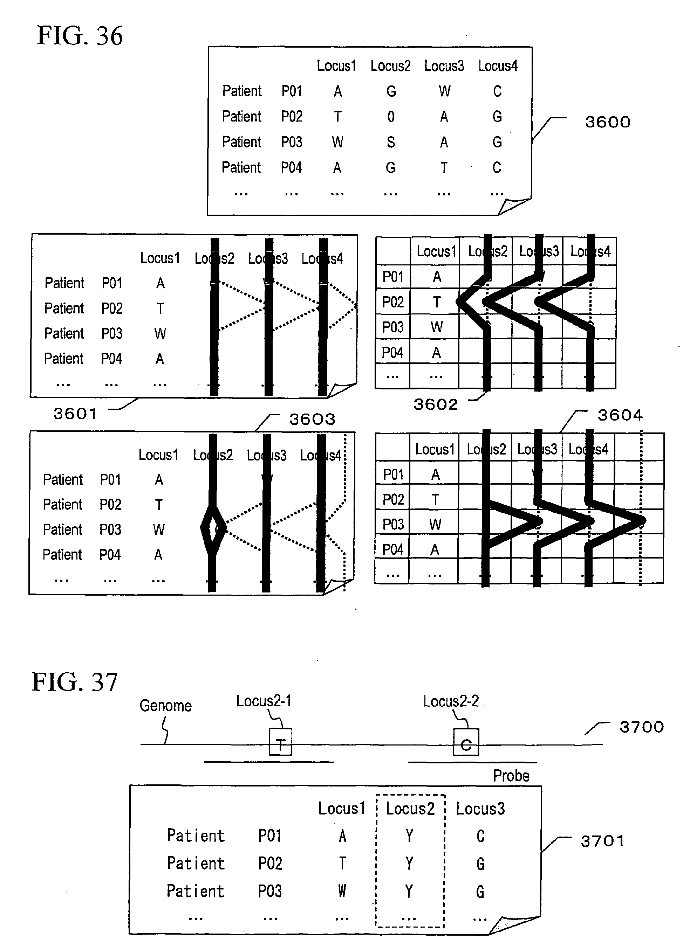

The second cause is that a heterozygous genotype has been described by mistake. In 3600 in FIG. 36, the individual P03 has alleles G and C at the locus 2. As shown in FIG. 34, a heterozygote comprised of G and C should be described as "S", but is now assumed to have been described as "K". Since K denotes a heterozygote comprised of G and T, it would be considered to have three alleles (G, C and T) though it actually has the two alleles (G and C).

The third cause is that missing data has been described as a blank character (a one-byte space, tab or the like) rather than "0". In FIG. 36, the individual P02 has no genotype at the locus 2. The missing data should be described as "0", but is now assumed to have been described as a one-byte space, as shown in 3601. Since a one-byte space means a break character in analysis programs for linkage disequilibrium, genotypic data at locus 2 and higher-numbered loci would shift one by one and be thus interpreted as the data shown in 3602. The loci 2 and 3 would be considered to have three or more alleles, respectively, according to the results of interpretation by the analysis program for linkage disequilibrium (the genotypic data connected to each other by the grey dotted line in 3601 and 3602), though they have only two alleles, respectively, according to the actual data (the genotypic data connected to each other by the grey bold line in 3601 and 3602). The individual P02 would have an unspecified genotype at the last locus 4.

The fourth cause is that a heterozygous genotype has been described by mistake. In FIG. 36, the genotype at the locus 2 in the individual P03 should be described as "S", but is now assumed to have been described as "G C" where the two alleles are separated by a one-byte space. In this case, genotypic data at locus 3 and higher-numbered loci would shift in a direction opposite to that shown in 3601 and be thus interpreted as the data shown in 3604. The loci 3 and 4 would be considered to have three or more alleles, respectively, according to the results of interpretation by the analysis program for linkage disequilibrium (the genotypic data connected to each other by the grey dotted line in 3603 and 3604), though they have only two alleles, respectively, according to the actual data (the genotypic data connected to each other by the grey bold line in 3603 and 3604). The individual P03 would have a specified genotype at the last locus (the locus name is unspecified).

In the cases of the third and fourth causes, it is not only difficult to associate the false description with the abnormal termination of the program, but also almost impossible to find out the false description among a large amount of the data including samples from 1,000 or more individuals and hundreds of loci. Such a situation is time-consuming to find out a cause for the problem and solve it.

### Error 8: loci lack of polymorphism are contained in the input data for a program where every locus is assumed to display polymorphism

When researchers use loci registered in a public data base such as JSNP, the loci are described as polymorphic in the data base, but may not be polymorphic (monomorphic) in the samples of the researchers. Some algorithms of linkage disequilibrium analysis are defined under the assumption that every locus used in the analysis displays polymorphism. For instance, a linkage disequilibrium measure, D' is determined by calculation using the frequencies of alleles in a divisor. Accordingly, the measure is not defined for a locus having an allele with zero frequency. If non-polymorphic loci are contained in the input data for such a program, the program could abnormally terminate on the way, or analysis results could be different from those intended even if the program can be executed.

### Error 9: in/del polymorphism is contained in the input data for a program where nothing other than A, T, G or C is assumed to appear in alleles

In this situation, the program could abnormally terminate on the way, or analysis results could be different from those intended even if the program can be executed.

### Error 10: an extraordinarily great number of individuals have the same heterozygous locus

To study genotypes experimentally, a short nucleotide sequence called a probe is provided for each locus in many cases. In SNP samples provided by JSNP or Applied Biosystems Inc., it may be expected that the probe is confirmed to react with only one location on the genome, but in SNP samples registered in a public data base such as dbSNP, which can be accessed by the general public, or in SNP samples provided by researchers on their own, the probe may react with two locations, though it is rare, as shown in 3700 of FIG. 37. If it happens, such experimental results is obtained as if nearly all individuals had a single locus 2 (a portion enclosed by a dotted line) displaying a heterozygote comprised of T and C, as shown in 3701, though neither locus 2-1 nor locus 2-2 actually displays polymorphism.

### Error 11: an extraordinarily great number of individuals have the same homozygous locus

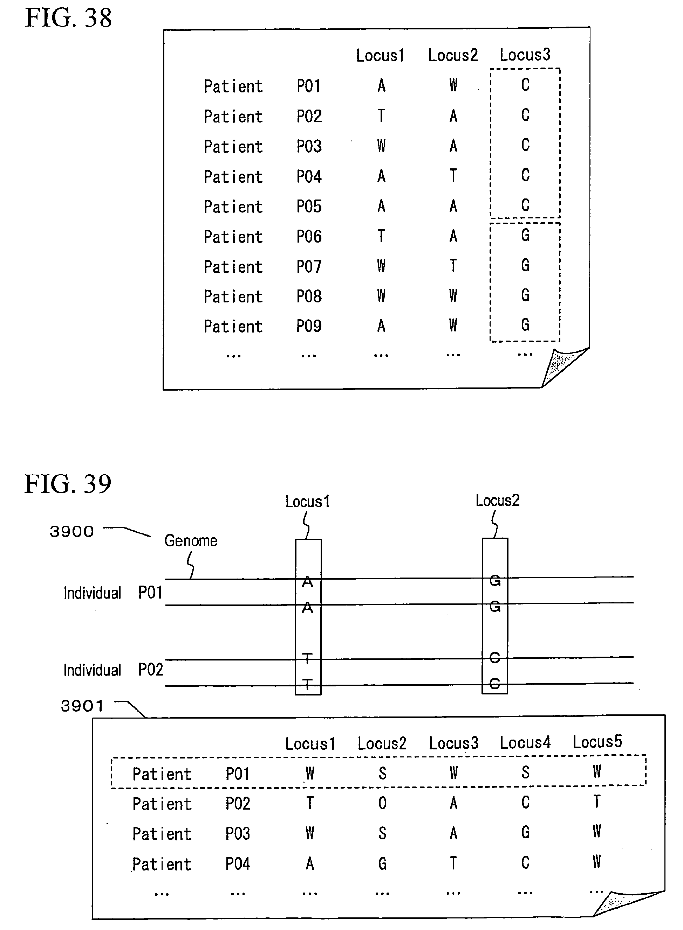

There are two conceivable causes. The first cause is that a sample population comprises many samples containing such homozygote samples. For diseases which may be caused by homozygous mutation at a higher risk than by heterozygous mutation, a patient population may be homozygote more frequently. The second cause is that the sample population is composed of two populations. For instance, there is now assumed to be a locus 3 where every individual of human race 1 has C and every individual of human race 2 has G. If the sample population comprises the two human races, the resultant data seem as if the locus displayed polymorphism, as shown in FIG. 38 though either of the races is not polymorphic. A sample population composed of two or more populations is not suitable for the analysis.

### Error 12: some individuals have an extraordinarily great number of heterozygous loci

There may happen a case where one sample is accidentally contaminated by a portion of another sample during the experiment. In the state shown in 3900 of FIG. 39, the DNA of the individual P02 is now assumed to have been incorporated into the DNA of the individual P01. As for the individual P01, the resultant data is observed as if the loci 1 and 2 had A and T, and G and C, respectively, as shown in 3901, though it is the fact that the loci 1 and 2 have only A and G, respectively, as allele. Therefore, the individual P01 would have the experimental result that there are heterozygotes in many gene loci.

### Error 13: some individuals have an extraordinarily great number of homozygous loci

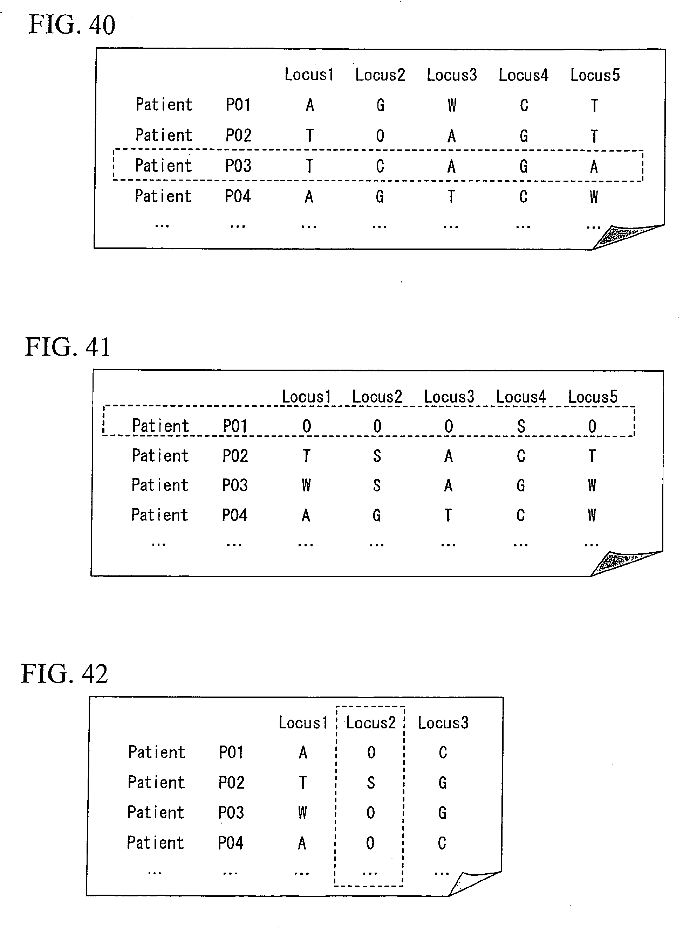

In a case shown in FIG. 40, the individual P03 is homozygous at every locus. Such an individual may be a special individual (for example, consanguineous marriages may have been made in the family line). In linkage disequilibrium analysis, it is postulated that samples have been chosen randomly from both a patient population and a healthy population. Consequently, it is often preferable to exclude this individual.

### Error 14: some individuals have many missing data

As shown in FIG. 41, many experimental failures may occasionally happen in a particular individual (P01 in this case) and produce many missing data. If it happens, accuracy of haplotype estimation would fall and/or a wider confidence interval. Accordingly, it is preferable to make both an analysis including the data of the individual P01 and an analysis excluding it.

### Error 15: some loci have many missing data

As shown in FIG. 42, many experimental failures may occasionally happen in a particular locus (locus 2 in this case) and produce many missing data. In addition, when genotype data from two or more research institutions are analyzed together, only one of the institutions is now assumed to have studied on locus 2 experimentally. If it happens, the data from the other institution will be treated as data having nothing but missing data for locus 2. In these cases, it is preferable to make both an analysis including the data for the locus 2 and an analysis excluding it, as in Error 14.

### Error 16: the sample population deviates from the Hardy-Weinberg equilibrium

When a population has a good number of individuals and has the conditions that: no individuals immigrate into a different population; random mating in population is made; and neither mutations nor natural selections occur, the population is said to be in Hardy-Weinberg equilibrium. If the sample population used in the analysis deviates from Hardy-Weinberg equilibrium, it will be doubtful if the samples have been taken randomly, and a suitable analysis could not be made.

### Error 17: some of the loci used in the analysis are extremely distant from the other loci

When the distance between the loci is very long, it is highly unlikely to think that the loci are in linkage disequilibrium (the loci are inherited as a bunch from the ancestor). Therefore, these loci should not be analyzed at once for linkage disequilibrium.

### Error 18: some loci have extremely rare alleles

In a search for pathogenic genes by statistical gene analysis, it is usually considered desirable to analyze only loci having a minor allele with a frequency of at least 5%, preferably of at least 10 to 30%. This limitation is set to prevent the power of statistic test from lowering by use of loci having alleles with an extremely low frequency. Accordingly, it is preferable to make both an analysis including the data for the locus and an analysis excluding them.

It is the object of the present invention to provide a data input support system which can preliminarily detect and remove such causes of errors as described above in making entries of genotype data for a program to execute linkage disequilibrium analysis or the like.

### SUMMARY OF THE INVENTION

As a result of every effort to solve the problem described above, the present inventors have now proposed a data input support system wherein, paying attention to limiting conditions characteristic of genotype input data and the statistical properties of the entire data set, the types of possible errors are preliminarily assumed, the input data are preprocessed to detect these errors, and the detected errors are associated with false descriptions causing them in order to report the results to the user. By means of such a data input support system, linkage disequilibrium analysis using appropriate data can be conducted efficiently, and the output of analysis results contrary to the user's intention can be avoided. More specifically, the following functions 1 to 15 will be used as means to correct the above errors 1 to 15, respectively.

Function 1: the system retains information as to if each analysis program needs the physical positions of loci as input data, and if an analysis program specified by a user needs the specified physical positions of loci, but they have not yet been specified in the input data, the system reports it.

Function 2-1: the system retains information as to if each analysis program assumes the arrangement of loci in order of their physical positions, and if an analysis program specified by a user assumes the arrangement of loci in order of their physical positions, but such arrangement is not provided in the input data, the system reports it.

Function 2-2: if Function 2-1 applies, the system produces a modified version of the input data having the loci rearranged.

Function 3: the system checks if the physical positions of loci overlap, and if they overlap, the system reports it.

Function 4-1: the system checks if loci having genotypes unspecified in every individual and loci having physical positions unspecified are present. If such a set of loci is present, the system checks if the loci have similar names, and if the loci have similar names, the system reports possible false descriptions of the names of the loci.

Function 4-2: if Function 4-1 applies, the system produces a modified version of the input data having the names of the loci made uniform into one of the names.

Function 5-1: the system checks if a symbol such as "*" (asterisk) is specified as genotype data, and if genotypes have such a symbol, the system reports possible false descriptions of the missing data.

Function 5-2: if Function 5-1 applies, the system produces a modified version of the input data having the descriptions of the genotypes replaced by "0" for missing data.

Function 5-3: the system checks if continuous form of two alleles such as AT are specified as genotype data, and if genotypes have such character strings, the system reports possible false descriptions of the heterozygous genotypes.

Function 5-4: if Function 5-3 applies, the system produces a modified version of the input data having the replaced descriptions of the heterozygous genotypes.

Function 5-5: the system checks if unexpected character strings such as "N" are specified as genotype data, and if genotypes have such character strings, the system reports it.

Function 6-1: the system retains information as to if each analysis program assumes the use of patients and healthy persons as input data, and if an analysis program specified by a user assumes the use of patients and healthy persons as input data, but the names of their populations are unspecified, the system reports it.

Function 6-2: the system checks if "Case" or "Control" is specified as population name, or an erroneously spelled name for "Patient" or "Normal" is specified where capital and/or small letters are wrongly used, and reports such a possible false description of "Patient" or "Normal".

Function 6-3: if Function 6-2 applies, the system produces a modified version of the input data having the descriptions of the population names replaced by "Patient" or "Normal".

Function 6-4: the system retains information as to if each analysis program assumes the use of patients and healthy persons as input data, and if an analysis program specified by a user assumes the use of patients and healthy persons, but an unexpected character string such as "Japanese" is specified as population name, the system reports it.

Function 7-1: the system retains information as to if each analysis program assumes the presence of two alleles at each locus and information as to what experimental technique is taken for each locus, and if an analysis program specified by a user assumes the presence of two alleles, or such an experimental technique is taken as can discriminate only two alleles, but loci with three or more alleles are actually present, the system reports it.

Function 7-2: if Function 7-1 applies, the system produces a modified version of the input data where those loci are excluded from the input data to be analyzed.

Function 7-3: if Function 7-1 applies, the system produces a modified version of the input data where the most frequent allele is combined with a third or higher-numbered most frequent allele in those loci.

Function 7-4: the system checks if there are loci having three or more alleles. If such loci are present, the system checks if both conditions described below are satisfied. If both of the conditions are satisfied, the system reports possible false descriptions of genotypes where the most frequent two of the alleles are heterozygous. 1) The most frequent two of the alleles are developed only as homozygotes, and there are no individuals having heterozygotes between the most frequent two of the alleles. 2) A third or higher-numbered most frequent allele is developed only as heterozygotes, and there are no individuals having homozygotes between the third and higher-numbered most frequent alleles.

Function 7-5: if Function 7-4 applies, the system produces a modified version of the input data having the heterozygous genotypes rewritten.

Function 7-6: the system checks if there is a locus having three or more alleles. If such a locus is present, the system checks if all of the four conditions described below are satisfied. If all of the four conditions are satisfied, the system reports possible descriptions of missing data as blank characters (a one-byte space, tab or the like). 1) A number of loci having three or more alleles appear which are more highly numbered than the above locus. 2) It is the same individual that has a third or higher-numbered most frequent allele at each locus having three or more alleles. 3) In the individual having a third or higher-numbered most frequent allele in common, the genotype at the last locus is not specified. 4) A third or higher-numbered most frequent allele at each locus having three or more alleles appears as a first or second most frequent allele at the next right locus.

Function 7-7: if Function 7-6 applies, the system produces a modified version of the input data having the descriptions of missing data replaced by "0".

Function 7-8: the system checks if there is a locus having three or more alleles. If such a locus is present, the system checks if all of the four conditions described below are satisfied. If all of the four conditions are satisfied, the system reports possible description of a heterozygous genotype by two alleles separated by a one-byte space. 1) A number of loci having three or more alleles appear which are more highly numbered than the above locus. 2) It is the same individual that has a third or higher-numbered most frequent allele at each locus having three or more alleles. 3) In the individual having a third or higher-numbered most frequent allele in common, the last locus with no specified locus name has a specified genotype. 4) A third or higher-numbered most frequent allele at each locus having three or more alleles appears as a first or second most frequent allele at the next left locus.

Function 7-9: if Function 7-8 applies, the system produces a modified version of the input data having the heterozygous genotype rewritten.

Function 7-10: the system checks if blank characters (a one-byte space, tab or the like) are irregularly used. If any of the following three conditions is satisfied, the system reports possible interpretation of the input data contrary to the intention of a user. 1) Two or more kinds of blank characters are used as break character for the input data. 2) Two or more blank characters appear in succession. 3) Such characters (a double-byte space or the like) as may be interpreted as either blank character or data are used.

In the IUB coding system, an individual identifier and locus data, or locus data to each other are assumed to be separated by a blank character (a one-byte space, tab or the like), typically a tab. However, since blank characters are not displayed on the screen by a usual text editor, two or more kinds of blank characters may be present one after another, or a double-byte space may be accidentally input in stead of a one-byte space, or an unnecessary blank character may be input at the end of a line. Furthermore, since a usual spreadsheet software interprets data by tab delimitation and displays each column of data in a vertical arrangement, a user may possibly not recognize that genotype data have been missed out, or described as a one-byte or double-byte space, or described as two alleles separated by a one-byte space. Error 7 described above can be securely prevented by utilizing Function 7-10 to report the irregular uses of blank characters.

Function 8-1: the system retains information as to if each analysis program assumes every locus to be polymorphic, and if an analysis program specified by a user assumes polymorphism in such a way, but some loci are monomorphic, the system reports it.

Function 8-2: if Function 8-1 applies, the system produces a modified version of the input data where the loci are excluded from the input data to be analyzed.

Function 9-1: the system retains information as to if each analysis program assumes nothing but A, T, G and C as allele, and if an analysis program specified by a user assumes nothing but A, T, G and C as allele, but some loci are in/del polymorphic, the system reports it.

Function 9-2: if Function 9-1 applies, the system produces a modified version of the input data where the in/del polymorphic loci are excluded.

Function 10-1: the system checks if there are loci heterozygous in extremely many individuals, and if such loci are present, the system reports possible reaction of probes for the loci at two or more locations on the genome.

Function 10-2: if Function 10-1 applies, the system produces a modified version of the input data where the loci are excluded from the input data to be analyzed.

Function 11: the system checks if there are loci homozygous in extremely many individuals, and if such loci are present, the system reports a possible presence of two or more populations in the sample population.

Function 12-1: the system checks if there are individuals having extremely many heterozygous loci, and if such individuals are present, the system reports a possible contamination.

Function 12-2: if Function 12-1 applies, the system produces a modified version of the input data where the individuals are excluded from the input data to be analyzed.

Function 13-1: the system checks if there are individuals having extremely many homozygous loci, and if such individuals are present, the system reports a possible peculiarity of the individuals.

Function 13-2: if Function 13-1 applies, the system produces a modified version of the input data where the individuals are excluded from the input data to be analyzed.

Function 14-1: the system checks if there are individuals having many missing data, and if such individuals are present, the system reports it.

Function 14-2: if Function 14-1 applies, the system produces a modified version of the input data where the individuals are excluded from the input data to be analyzed.

Function 15: the system lists and displays both the items reported using Functions 1 to 14-2 described above and the items for which modified versions of the input data have been produced.

Errors 1 to 14 can be prevented by use of Functions 1 to 14-2, respectively. In addition, Errors 15, 16, 17 and 18 can be dealt with by conventional techniques such as Haploview and Varia described above.

The present invention provides, as a system having the above Functions 1 to 15, a data input support system to inspect genotype data which are input into a program for linkage disequilibrium analysis, wherein the system comprises a storage section for retaining error types for genotype data corresponding to the program for linkage disequilibrium analysis, an error detection section for checking the input genotype data for the error types and detecting errors, and an error report/display section for displaying the report of the detected errors.

In the inventive data input support system, the error types are characterized by comprising the error that the input genotype data has no data on the physical positions of loci, opposed to a program for linkage disequilibrium analysis requiring genotype data on the physical positions of the loci. This provides the above Function 1.

In the inventive data input support system, the error types are characterized by comprising the error that in the input genotype data, the loci are not arranged in order of their physical positions, opposed to a program for linkage disequilibrium analysis corresponding only to genotype data where the loci are arranged in order of their physical positions. This provides the above Function 2 (branch number is omitted, and it will be omitted hereafter).

In the inventive data input support system, the error types are characterized by comprising the error that the input genotype data has the physical positions of loci overlapped. This provides the above Function 3.

In the inventive data input support system, the error types are characterized by comprising the error that the input genotype data contains loci having genotypes unspecified and loci having physical positions unspecified. This provides the above Function 4.

In the inventive data input support system, the error types are characterized by comprising the error that in the input genotype data, some symbols denoting a homozygote, a heterozygote or missing data are different from those defined by the program for linkage disequilibrium analysis. This provides the above Function 5.

In the inventive data input support system, the error types are characterized by comprising the error that in the input genotype data, neither a patient population nor a healthy population is specified according to the definitions made by a program for linkage disequilibrium analysis, opposed to the program for linkage disequilibrium analysis requiring the genotype data of both patients and healthy persons. This provides the above Function 6.

In the inventive data input support system, the error types are characterized by comprising the error that the input genotype data contains loci having three or more alleles, opposed to a program for linkage disequilibrium analysis defining that at most two alleles are present in a locus. This provides the above Function 7.

In the inventive data input support system, the error types are characterized by comprising the error that the input genotype data contains any of the following descriptions:
1) at least two different blank characters are used as break character for the input data;
2) at least two blank characters appear in succession; and
3) characters are used which can be interpreted as either blank character or genotype data depending on the type of a program for linkage disequilibrium analysis.
This provides the above Function 7.

In the inventive data input support system, the error types are characterized by comprising the error that the input genotype data contains monomorphic loci, opposed to a program for linkage disequilibrium analysis defining that every locus is polymorphic. This provides the above Function 8.

In the inventive data input support system, the error types are characterized by comprising the error that the input genotype data contains in/del polymorphic loci, opposed to a program for linkage disequilibrium analysis defining that nothing but A, T, G or C appears as allele. This provides the above Function 9.

In the inventive data input support system, the error types are characterized by comprising the error that the input genotype data contains a higher level of individuals where the locus is heterozygous than a predetermined level, or a higher level of individuals where the locus is homozygous than a predetermined level. Herein, the predetermined level may be selected from a rate of number of individuals, a P value in a statistical test, or the like. This provides the above Functions 10 and 11.

In the inventive data input support system, the error types are characterized by comprising the error that the input genotype data contains individuals having a higher level of heterozygous loci than a predetermined level, or individuals having a higher level of homozygous loci than a predetermined level. Herein, the predetermined level may be selected from a rate of number of individuals, a P value in a statistical test, or the like. This provides the above Functions 12 and 13.

In the inventive data input support system, the error types are characterized by comprising the error that the input genotype data contains individuals having a higher level of missing data than a predetermined level. Herein, the predetermined level used may be a rate of number of individuals or the like. This provides the above Function 14.

In the inventive data input support system, the above Function 7 has further characteristics as described below.

If genotype data contain loci having three or more alleles, and both conditions described below are satisfied, the error report/display section displays a report on possible false descriptions in the input genotype data of genotypes where the most frequent two of the three or more alleles are heterozygous.
1) In the input genotype data, there are no individuals having heterozygote comprised of the most frequent two of the three or more alleles.
2) In the input genotype data, there are no individuals having homozygosis between the third and higher-numbered most frequent ones of the three or more alleles.

If genotype data contain a locus having three or more alleles, and the four conditions described below are satisfied, the error report/display section displays a report on possible false descriptions in the input genotype data of missing data.
1) In the input genotype data, a certain or more number of loci having three or more alleles is present subsequent to the locus having three or more alleles.
2) In the input genotype data, the same individual has a third or higher-numbered most frequent allele of the three or more alleles at two or more loci.
3) In the input genotype data, in the individual applying to the above 2), the genotype at the last locus is not specified.
4) In the input genotype data, a third or higher-numbered most frequent allele at a locus having three or more alleles appears as a first or second most frequent allele at the next right locus.

If genotype data contain a locus having three or more alleles, and the four conditions described below are satisfied, the error report/display section displays a report on possible false description in the input genotype data of a heterozygous genotype.
1) In the input genotype data, a certain or more number of loci having three or more alleles is present subsequent to the locus having three or more alleles.
2) In the input genotype data, the same individual has a third or higher-numbered most frequent allele of the three or more alleles at two or more loci.
3) In the input genotype data, in the individual applying to the above 2), the genotype at the last locus is specified.
4) In the input genotype data, a third or higher-numbered most frequent allele at a locus having three or more alleles appears as a first or second most frequent allele at the next left locus.

In addition, the inventive data input support system is characterized by also comprising error correction means to accept an input for correcting the reported error in the input genotype data and correct the input genotype data based on the input.

In the inventive data input support system, the error correction means is characterized by accepting a correction input by which for the locus having three or more alleles, a third or higher-numbered most frequent allele of the three or more alleles is rewritten into a first or higher-numbered most frequent allele, and thereby correcting the genotype data in such a manner.

The inventive data input support system is characterized by further comprising means to display as a list the content of errors reported by the error report/display section as well as the content of corrections for the genotype data by the error correction means.

According to the present invention, as described above, various errors can be detected which are contained in data to be input for a program for linkage disequilibrium analysis or the like, and the errors can be associated with false descriptions resulting in the errors to display the results. In this way, the linkage disequilibrium analysis can be conducted efficiently using appropriate data, and the output of analysis results contrary to the intention of a user can be avoided.

### BRIEF DESCRIPTION OF THE DRAWINGS

FIG. 1 is a functional block diagram outlining the system configuration of the inventive support system for interpretation of genetic data;
FIG. 2 illustrates a data composition of program data stored in the data memory of the inventive support system for interpretation of genetic data;
FIG. 3 illustrates a data composition of input data stored in the data memory of the inventive support system for interpretation of genetic data;
FIG. 4 is a flow chart outlining processing in the inventive support system for interpretation of genetic data;
FIG. 5 is a flow chart showing a detailed flow of the processing of detecting and reporting errors, accepting user input, and producing a modified version of the input data in the inventive support system for interpretation of genetic data;
FIG. 6 is a flow chart showing a detailed flow of the processing of checking and reporting if an unexpected genotype is present in the inventive support system for interpretation of genetic data;
FIG. 7 is a flow chart showing a detailed flow of the processing of checking and reporting if a population name is erroneous in the inventive support system for interpretation of genetic data;
FIG. 8 is a flow chart showing a detailed flow of the processing of checking and reporting if a locus having three or more alleles is present in the inventive support system for interpretation of genetic data;
FIG. 9 illustrates a display screen made by a physical position specification report processing section at step 500 in the flow chart shown in FIG. 5;
FIG. 10 illustrates a display screen made by a physical position order report processing section at step 501 in the flow chart shown in FIG. 5;
FIG. 11 illustrates a display screen made by a physical positions overlap report processing section at step 502 in the flow chart shown in FIG. 5;
FIG. 12 illustrates a display screen made by a similar locus name report processing section at step 503 in the flow chart shown in FIG. 5;
FIG. 13 illustrates a display screen made by a symbol genotype report processing section at step 600 in the flow chart shown in FIG. 6;
FIG. 14 illustrates a display screen made by a character string genotype report processing section at step 601 in the flow chart shown in FIG. 6;
FIG. 15 illustrates a display screen made by an unexpected genotype report processing section at step 602 in the flow chart shown in FIG. 6;
FIG. 16 illustrates a display screen made by a specified population name report processing section at step 700 in the flow chart shown in FIG. 7;
FIG. 17 illustrates a display screen made by a falsely described population name report processing section at step 701 in the flow chart shown in FIG. 7;
FIG. 18 illustrates a display screen made by an unexpected population name report processing section at step 702 in the flow chart shown in FIG. 7;
FIG. 19 illustrates a display screen made by a multiple alleles report processing section at step 803 in the flow chart shown in FIG. 8;
FIG. 20 illustrates a display screen made by a falsely described heterozygotes report processing section at step 802 in the flow chart shown in FIG. 8;
FIG. 21 illustrates a display screen made by a missing blank report processing section at step 800 in the flow chart shown in FIG. 8;
FIG. 22 illustrates a display screen made by a heterozygosis blank report processing section at step 801 in the flow chart shown in FIG. 8;
FIG. 23 illustrates a display screen made by an irregular blank character report processing section at step 804 in the flow chart shown in FIG. 8;
FIG. 24 illustrates a display screen made by a monomorphism report processing section at step 507 in the flow chart shown in FIG. 5;
FIG. 25 illustrates a display screen made by an in/del report processing section at step 508 in the flow chart shown in FIG. 5;
FIG. 26 illustrates a display screen made by a dual site reaction report processing section at step 509 in the flow chart shown in FIG. 5;
FIG. 27 illustrates a display screen made by a plural populations report processing section at step 510 in the flow chart shown in FIG. 5;
FIG. 28 illustrates a display screen made by contamination report processing section at step 511 in the flow chart shown in FIG. 5;
FIG. 29 illustrates a display screen made by a special individual report processing section at step 512 in the flow chart shown in FIG. 5;
FIG. 30 illustrates a display screen made by a missing individual report processing section at step 513 in the flow chart shown in FIG. 5;
FIG. 31 illustrates a display screen made by a reported/corrected items display processing section at step 514 in the flow chart shown in FIG. 5;
FIG. 32 illustrates SNP appearing on the genome;
FIG. 33 illustrates the format of an input file having genotype data described to enter them into a program for linkage disequilibrium analysis;
FIG. 34 illustrates IUB codes;
FIG. 35 illustrates the format of an input file having the physical position of each locus described to enter it into a program for linkage disequilibrium analysis;
FIG. 36 illustrates some cases where only two alleles are actually present, but three or more alleles are misjudged to be present;
FIG. 37 illustrates a case where a probe reacts with two locations on the genome;
FIG. 38 illustrates a case where a sample population is a combination of two different populations;
FIG. 39 illustrates a case where contamination from a different sample has occurred;
FIG. 40 illustrates a special individual;
FIG. 41 illustrates an individual having many missing data; and
FIG. 42 illustrates a locus having many missing data.

### DETAILED DESCRIPTION OF THE PREFERRED EMBODIMENTS

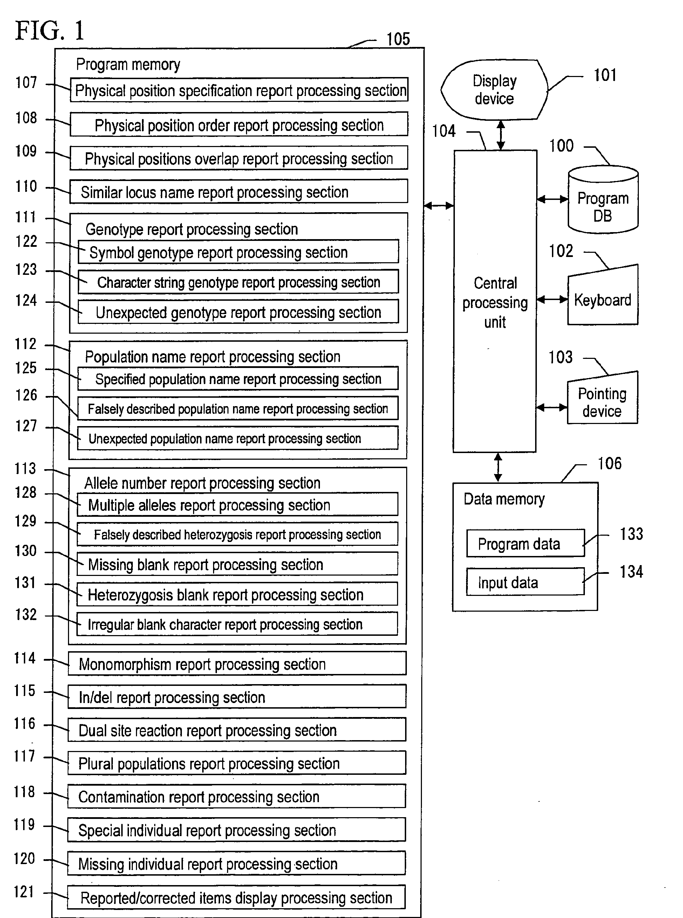

The best embodiment to carry out the inventive data input support system for gene analysis will be described below in detail referring to the appended drawings. FIGS. 1 to 31 illustrate the embodiment of the present invention, wherein a portion with an identical symbol represents the same matter and the basic constitution and operation are the same through the figures.

### Configuration of genotype data input support system

FIG. 1 shows a functional block diagram outlining the internal configuration of a genotype data input support system constructed in an embodiment of the present invention. The genotype data input support system comprises a program DB 100 where the features of various programs used in statistical gene analysis are saved, a display device 101 for displaying input data and supported interpretation results therefor, a key board 102 and a pointing device 103 such as a mouse for operation such as selection of individuals or loci from the displayed data or the like, a CPU 104 for carrying out necessary arithmetic processing, control processing and the like, a program memory 105 for storing the programs necessary to processing in the CPU 104, and a data memory 106 for storing data necessary to processing in the CPU 104.

The program memory 105 contains: a specified physical position report processing section 107 for execution of the above Function 1; a physical position order report processing section 108 for execution of Functions 2-1 and 2-2; a physical positions overlap report processing section 109 for execution of Function 3; a similar locus name report processing section 110 for execution of Functions 4-1 and 4-2; a genotype report processing section 111 for execution of Functions 5-1, 5-2, 5-3, 5-4 and 5-5; a population name report processing section 112 for execution of Functions 6-1, 6-2, 6-3 and 6-4; an allele number report processing section 113 for execution of Functions 7-1, 7-2, 7-3, 7-4, 7-5, 7-6, 7-7, 7-8, 7-9 and 7-10; a monomorphism report processing section 114 for execution of Functions 8-1 and 8-2; an in/del report processing section 115 for execution of Functions 9-1 and 9-2; a dual site reaction report processing section 116 for execution of Functions 10-1 and 10-2; a plural populations report processing section 117 for execution of Function 11; a contamination report processing section 118 for execution of Functions 12-1 and 12-2; a special individual report processing section 119 for execution of Functions 13-1 and 13-2; a missing individual report processing section 120 for execution of Functions 14-1 and 14-2; and a reported/corrected items display processing section 121 for execution of Function 15. Additionally, the genotype report processing section 111 comprises a symbol genotype report processing section 122 for execution of the above Functions 5-1 and 5-2, a character string genotype report processing section 123 for execution of Functions 5-3 and 5-4, and an unexpected genotype report processing section 124 for execution of Function 5-5; the population name report processing section 112 comprises a specified population name report processing section 125 for execution of the above Function 6-1, a falsely described population name report processing section 126 for execution of Functions 6-2 and 6-3, and an unexpected population name report processing section 127 for execution of Function 6-4; and the allele number report processing section 113 comprises a multiple alleles report processing section 128 for execution of the above Functions 7-1, 7-2 and 7-3, a falsely described heterozygosis report processing section 129 for execution of Functions 7-4 and 7-5, a missing blank report processing section 130 for execution of Functions 7-6 and 7-7, a heterozygosis blank report processing section 131 for execution of Functions 7-8 and 7-9, and an irregular blank character report processing section 132 for execution of Function 7-10.

The data memory 106 comprises program data 133 containing the features of programs used in statistical gene analysis and input data 134 used as input data for the programs.

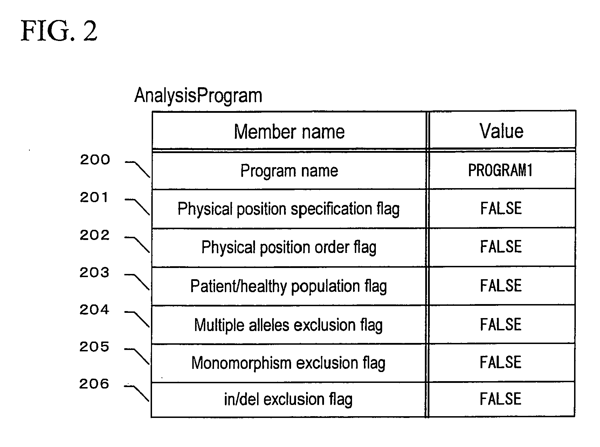

FIG. 2 shows the data structure of the program data 133 contained in the data memory 106. The data structure called AnalysisProgram comprises: a program name 200; a physical position specification flag 201 indicating if the physical positions of loci are required as input data; a physical position order flag 202 indicating if the loci are assumed to be arranged in the order of their physical positions; a patient/healthy population flag 203 indicating if both patients and healthy persons are assumed to be used; a multiple alleles exclusion flag 204 indicating if two alleles are assumed in each locus; a monomorphism exclusion flag 205 indicating if every locus is assumed to be polymorphic; and an in/del exclusion flag 206 indicating if nothing but A, T, G or C is assumed to appear as allele.

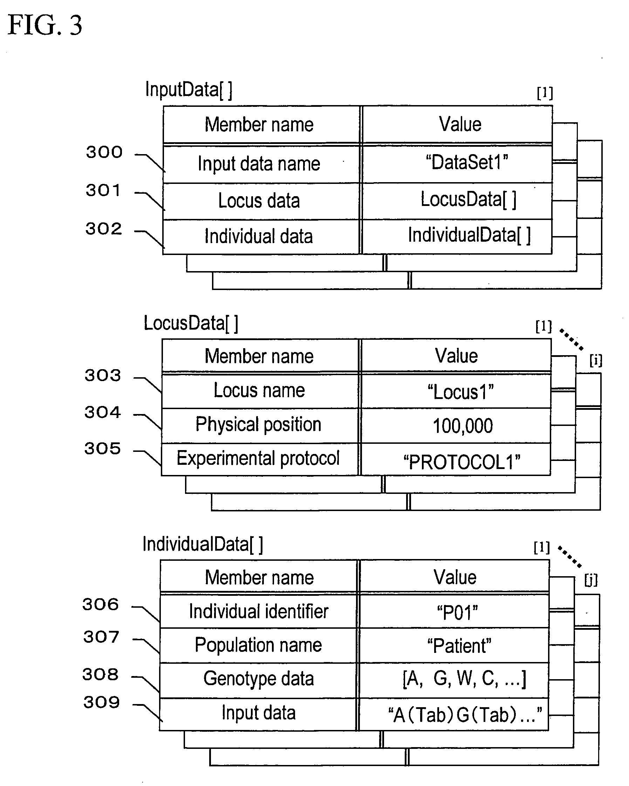

FIG. 3 shows the data structure of the input data 134 contained in the data memory 106. Hereinafter, unspecified data items will have a null value. The data structure called InputData comprises input data name 300, locus data 301 and individual data 302. The locus data 301 retains the data in the arrangement of a data structure called LocusData as described below. The individual data 302 retains the data in the arrangement of a data structure called IndividualData as described below.

The data structure LocusData comprises each locus name 303, its physical position 304 and an experimental protocol 305 used to determine the genotype at each locus for the number of loci, integer i.

The data structure IndividualData comprises: an individual identifier 306 for each individual; a population name 307 indicating the name of the population to which the individual belongs; a genotype data 308 indicating respective genotypes which the individual has at respective loci; and an original character string 309 in the input data, for the number of individual samples, integer j. The genotype data 308 represents an array for storing genotype data interpreted by separating the input data 309 into compartments with blank characters, and has the number of elements equal to the number of elements, integer i, in the locus data 301.

### Operation of genotype data input support system

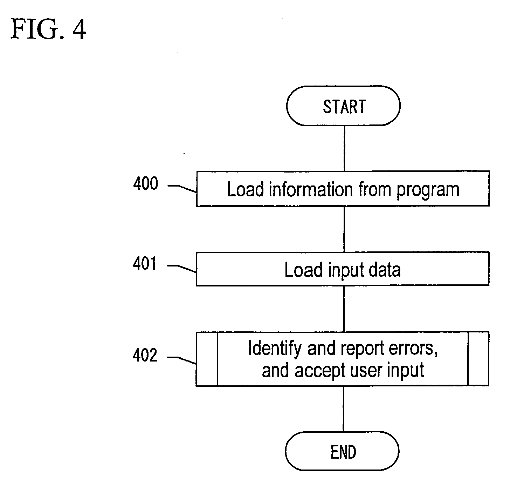

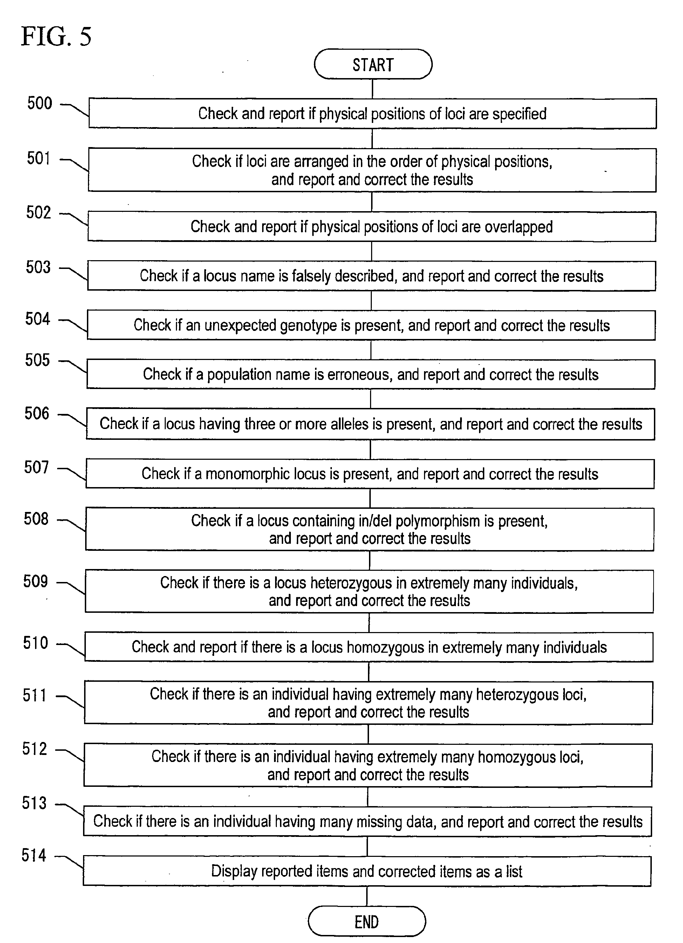

Next, processings executed in the genotype data input support system of the present embodiment will be now described which system is configured as described above. FIG. 4 shows a flow chart illustrating the processing flow in the genotype data input support system. In FIG. 4, data corresponding to a program specified by a user are first loaded from the program DB 100 (step 400). The data loaded here are retained as the program data 133 in the data memory 106. Input data used for the program and each experimental protocol for each locus are then loaded (step 401). The data loaded here are retained as the input data 134 in the data memory 106. Thereafter, errors in the input data are detected and reported, and user input is accepted to produce a modified version of the input data (step 402). These processings are executed using the processing sections 107 to 132 contained in the program memory 105, which will be described in detail referring to FIG. 5.

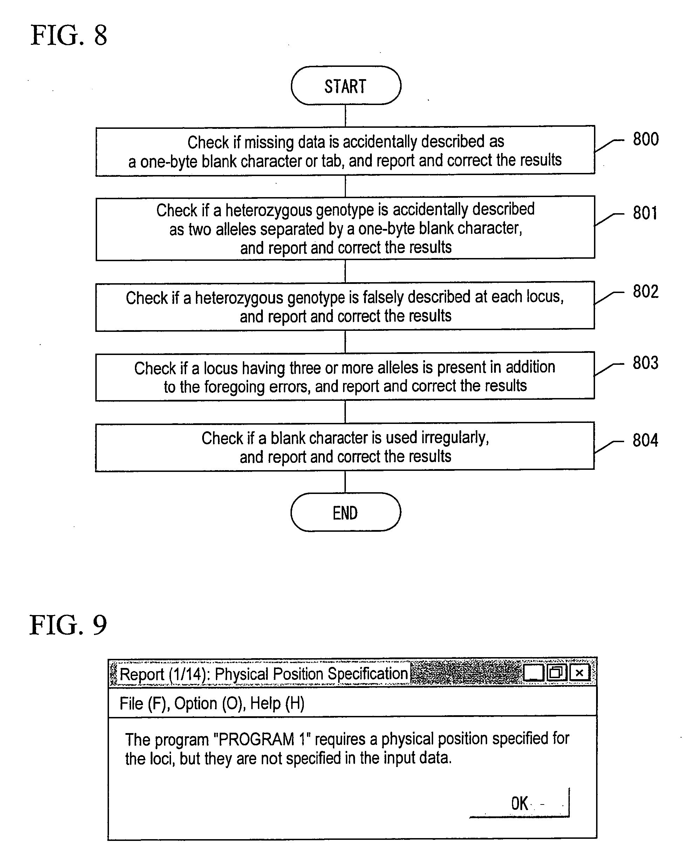

Next, the processing for checking and reporting if there are errors in the input data, and accepting user input, which is executed in step 402 in FIG. 4, will be detailed referring to a detailed flow chart shown in FIG. 5. First of all, it is checked and reported if the physical positions of loci are specified, using the specified physical position report processing section 107 (step 500). If the physical position specification flag 201 in the program data 133 is TRUE, and the physical position 304 of the locus data 301 in the input data 134 is not specified, an error is judged to be present and it is displayed on the screen as shown in FIG. 9.

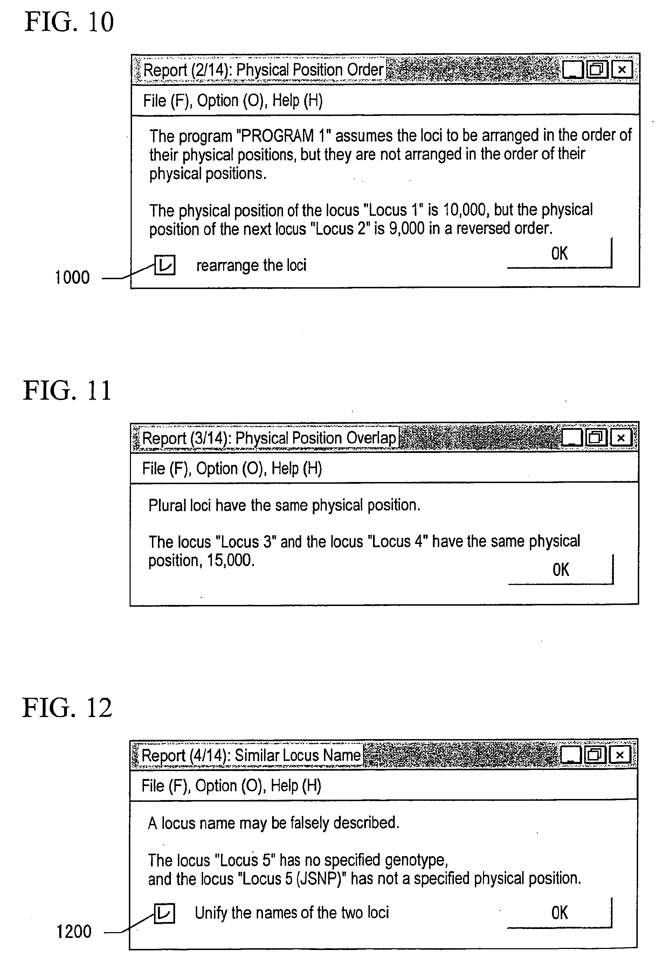

Next, it is checked if the input loci are arranged in the order of their physical positions, and the results are reported and corrected (step 501), using the physical position order report processing section 108. If the physical position order flag 202 in the program data 133 is TRUE, the physical position 304 of the locus data 301 in the input data 134 is investigated one after another. If some specified physical positions present a reversed magnitude correlation, an error is judged to be present and it is displayed on the screen as shown in FIG. 10. If the user ticks 1000, the data on the relevant two loci in the locus data 301, the genotype data 308, and the input data 309 are exchanged to produce a modified version of the input data.

Next, it is checked and reported if the physical positions of the loci are overlapped, using the physical positions overlap reporting/processing section 109 (step 502). The physical position 304 of the locus data 301 in the input data 134 is investigated one after another, and if some of the physical positions have the same number, an error is judged to be present and it is displayed on the screen as shown in FIG. 11.

Next, it is checked if a locus name is falsely described, and the results are reported and corrected (step 503), using the similar locus name report processing section 110. As described in the above Function 4-1, it is checked if there is a locus in which the genotype data 308 in the input data 134 are unspecified in every individual and there is a locus in which the physical position 304 is unspecified. If such a set of loci is present, and the loci have similar names, an error is judged to be present and it is displayed on the screen as shown in FIG. 12. If the user ticks 1100, the following operation is executed to produce a modified version of the input data. The physical position 304 of a locus having its genotype data 308 unspecified is transcribed for the other locus having its physical position 304 unspecified. Thereafter, the data on the locus having its genotype data 308 unspecified is deleted from the locus data 301, the genotype data 308, and the input data 309.

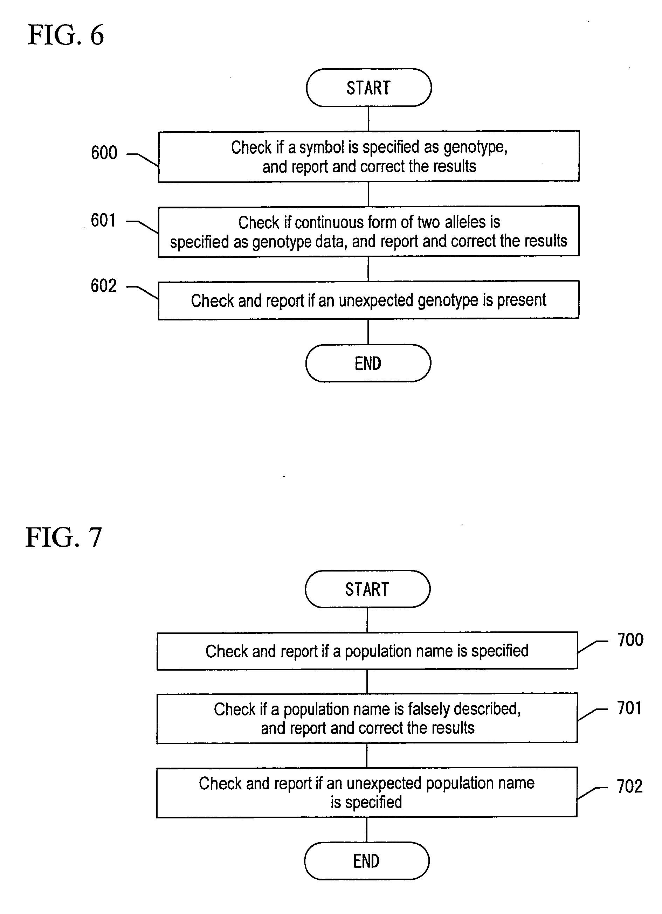

Next, it is checked if an unexpected genotype is present, and the results are reported and corrected (step 504), using the genotype reporting/processing section 111. This processing will be described in detail referring to FIG. 6.

Next, it is checked if a population name is erroneous, and the results are reported and corrected (step 505), using the population name reporting/processing section 112. This processing will be described in detail referring to FIG. 7.

Next, it is checked if a locus having three or more alleles is present, and the results are reported and corrected (step 506), using the allele number reporting/processing section 113. This processing will be described in detail referring to FIG. 8.

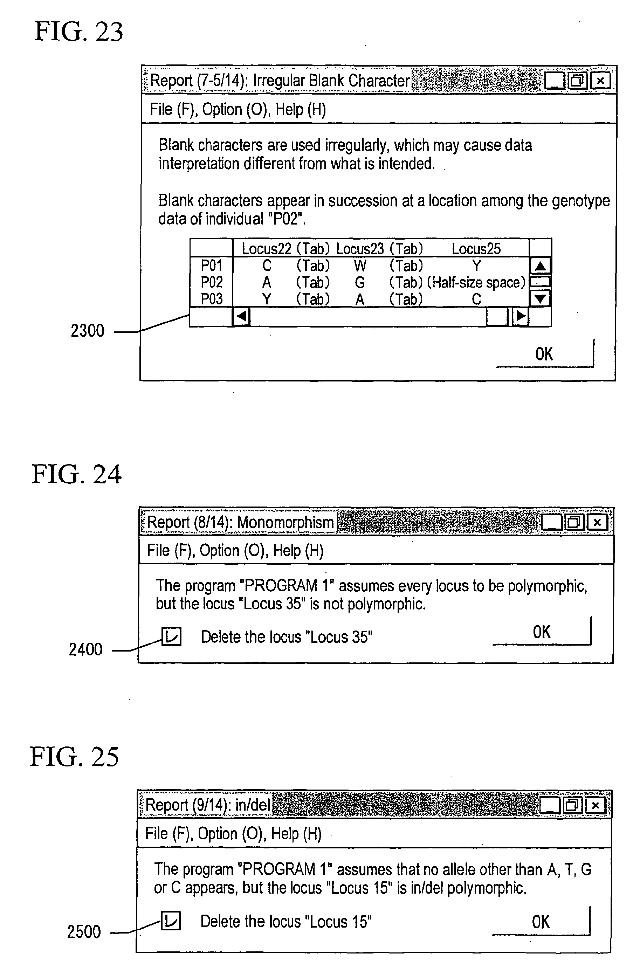

Next, it is checked if a monomorphic locus is present, and the results are reported and corrected (step 507), using the monomorphism reporting/processing section 114. If the monomorphism exclusion flag 205 in the program data 133 is TRUE, and the genotype data 308 in the input data 134 is not polymorphic, an error is judged to be present and it is displayed on the screen as shown in FIG. 24. If the user ticks 2400, the data on the relevant locus is deleted from the locus data 301, the genotype data 308, and the input data 309 to produce a modified version of the input data.

Next, it is checked if a locus containing in/del polymorphism is present, and the results are reported and corrected (step 508), using the in/del reporting/processing section 115. If the in/del exclusion flag 206 in the program data 133 is TRUE, and the genotype data 308 in the input data 134 is in/del polymorphic, an error is judged to be present and it is displayed on the screen as shown in FIG. 25. If the user ticks 2500, the data on the relevant locus is deleted from the locus data 301, the genotype data 308, and the input data 309 to produce a modified version of the input data.

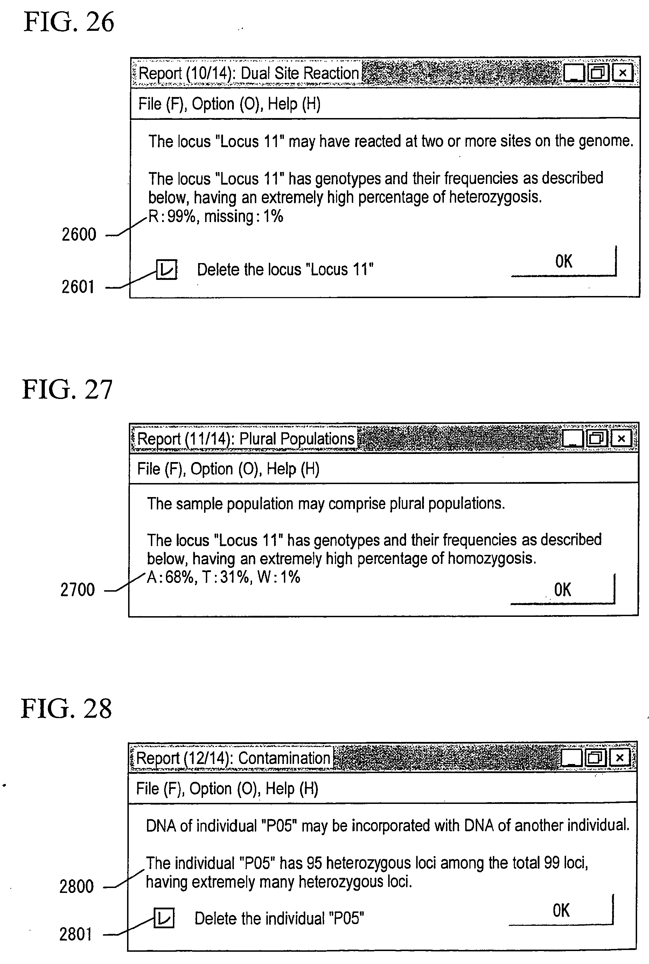

Next, it is checked if there is a locus heterozygous in extremely many individuals, and the results are reported and corrected (step 509), using the dual site reaction reporting/processing section 116. For each locus, the number rate of individuals having the heterozygous locus in the total individuals (heterozygosity), the occurrence probability of the locus with an observed heterozygosity (P value in the Hardy-Weinberg equilibrium test) or the like is used to evaluate the abundance of individuals heterozygous at the locus. If there is a locus heterozygous in extremely many individuals, it is displayed on the screen as shown in FIG. 26. The numeral 2600 in the screen display shows the genotype frequency for the locus summarized from the genotype data 308 for each individual. If the user ticks 2601, the data on the relevant locus is deleted from the locus data 301, the genotype data 308, and the input data 309 to produce a modified version of the input data.

Next, it is checked and reported if there is a locus homozygous in extremely many individuals (step 510), using the plural populations report processing section 117. For each locus, the number rate (homozygosity) of individuals having the homozygous locus in the total individuals, the occurrence probability (P value in the Hardy-Weinberg equilibrium test) of the locus with an observed homozygosity or the like is used to evaluate the abundance of individuals homozygous at the locus. If there is a locus homozygous in extremely many individuals, it is displayed on the screen as shown in FIG. 27. The numeral 2700 in the screen display shows the genotype frequency for the locus summarized from the genotype data 308 for each individual.

Next, it is checked if there is an individual having extremely many heterozygous loci, and the results are reported and corrected (step 511), using the contamination report processing section 118. For each individual, the number rate of the heterozygous loci in the total loci, the occurrence probability (P value) of the individual with an observed number rate or the like is used to evaluate the abundance of heterozygous loci. If there is an individual having extremely many heterozygous loci, it is displayed on the screen as shown in FIG. 28. The numeral 2800 in the screen display shows the number rate of heterozygous loci summarized from the genotype data 308. If the user ticks 2801, the data on the relevant individual is deleted from the individual data 302 to produce a modified version of the input data.

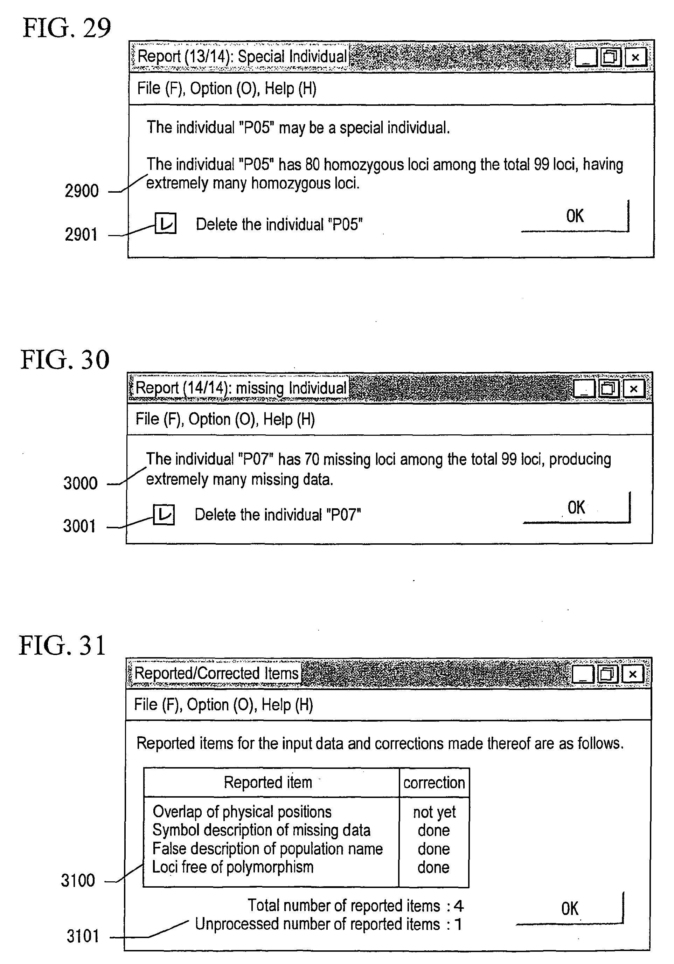

Next, it is checked if there is an individual having extremely many homozygous loci, and the results are reported and corrected (step 512), using the special individual reporting/processing section 119. For each individual, the number rate of the homozygous loci in the total loci, the occurrence probability (P value) of the individual with an observed number rate or the like is used to evaluate the abundance of homozygous loci. If there is an individual having extremely many homozygous loci, it is displayed on the screen as shown in FIG. 29. The numeral 2900 in the screen display shows the number rate of homozygous loci summarized from the genotype data 308. If the user ticks 2901, the data on the relevant individual is deleted from the individual data 302 to produce a modified version of the input data.

Next, it is checked if there is an individual having many missing data, and the results are reported and corrected (step 513), using the missing individual reporting/processing section 120. The number rate of the missing data in the total loci is used to evaluate the abundance of missing data. If there are far more missing data than a predetermined reference level, it is displayed on the screen as shown in FIG. 30. The numeral 3000 in the screen display shows the number rate of missing data summarized from the genotype data 308. If the user ticks 3001, the data on the relevant individual is deleted from the individual data 302 to produce a modified version of the input data.

Next, the reported items and items for each of which a modified version of the input data was produced in steps 500 to 513 are listed up and displayed on the screen as shown in FIG. 31 (step 514), using the reported/corrected items display processing section 121. The numeral 3100 in the screen display shows an outline of the respective reported items and if they were corrected, respectively. The numeral 3101 in the screen display shows the number of reported items and the number of reported items for each of which, however, a modified version of the input data was not produced.

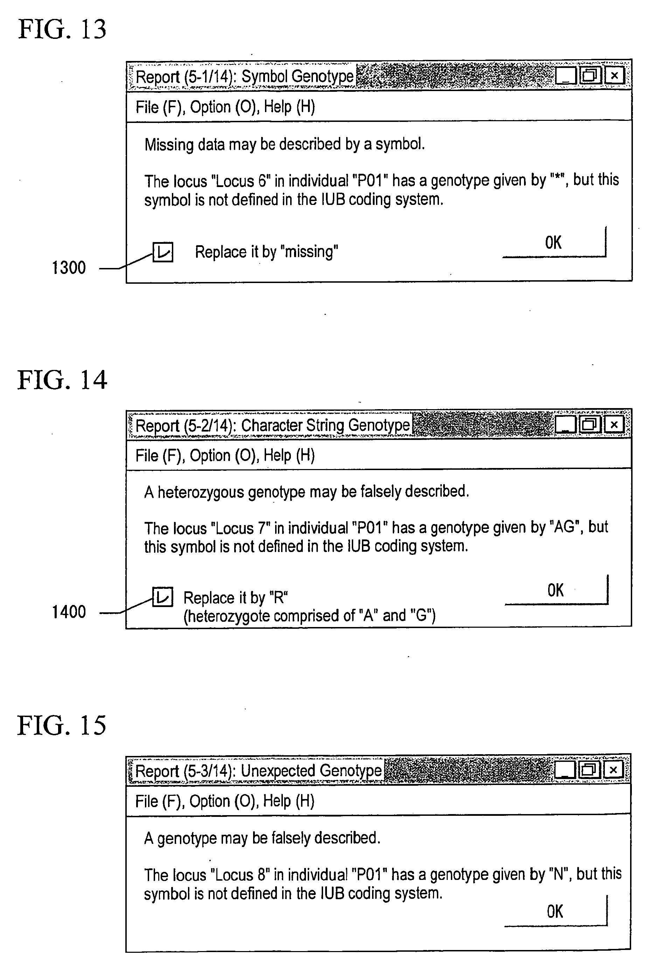

Next, the processing for checking if there is an unexpected genotype, and reporting and correcting the results, which is executed in step 504 in FIG. 5, will be detailed referring to a detailed flow chart shown in FIG. 6. It is first checked if a symbol such as "*" (asterisk) is specified as genotype, and the results are reported and corrected (step 600), using the symbol genotype report processing section 122. If there is such a genotype, it is displayed on the screen as shown in FIG. 13. If the user ticks 1300, "0" is entered in the relevant element in the genotype data 308 and the input data 309 to produce a modified version of the input data.

Next, it is checked if a character string of two alleles is specified as genotype data, and the results are reported and corrected (step 601), using the character string genotype report processing section 123. If there is such a genotype, it is displayed on the screen as shown in FIG. 14. If the user ticks 1400, a correct heterozygous genotype is entered in the relevant element in the genotype data 308 and the input data 309 to produce a modified version of the input data.

Next, it is checked and reported if an unexpected character string is specified as genotype data (step 602), using the unexpected genotype report processing section 124. If there is such a genotype, it is displayed on the screen as shown in FIG. 15.

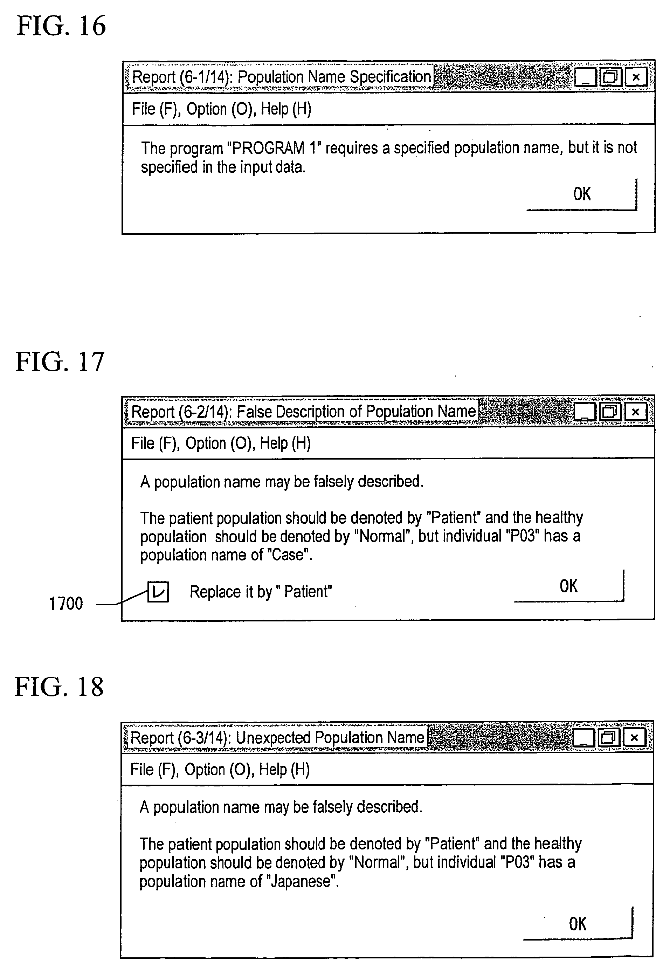

Next, the processing for checking if a population name is erroneous, and reporting and correcting the results, which is executed in step 505 in FIG. 5, will be detailed referring to a detailed flow chart shown in FIG. 7. It is first checked and reported if a population name is specified (step 700), using the specified population name report processing section 125. If the patient/healthy population flag 203 in the program data 133 is TRUE, and the population name 307 of the individual data 302 in the input data 134 is not specified, an error is judged to be present and it is displayed on the screen as shown in FIG. 16.

Next, it is checked if "Case" or "Control" is specified as population name, or an erroneously spelled name for "Patient" or "Normal" is specified where capital and/or small letters are wrongly used, and the results are reported and corrected (step 701), using the falsely described population name reporting/processing section 126. If there is an individual with such a population name specified, it is displayed on the screen as shown in FIG. 17. If the user ticks 1700, a correct population name is entered in the population name 307 to produce a modified version of the input data.

Next, it is checked and reported if an unexpected character string is specified as population name (step 702), using the unexpected population name report processing section 127. If there is an individual with such a population name specified, it is displayed on the screen as shown in FIG. 18.

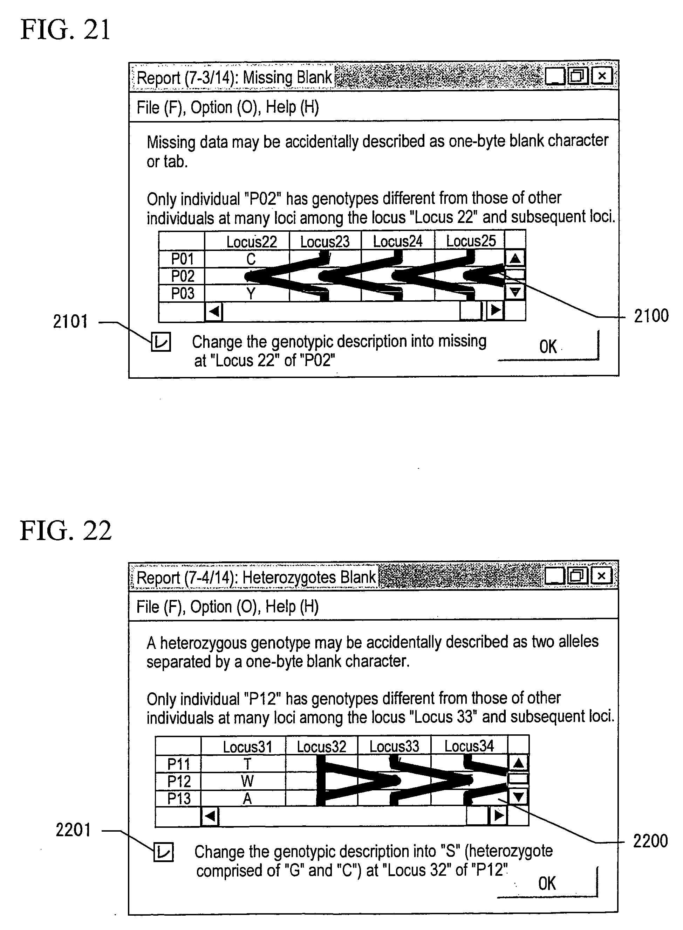

Next, the processing for checking if there is a locus having three or more alleles, and reporting and correcting the results, which is executed in step 506 in FIG. 5, will be detailed referring to a detailed flow chart shown in FIG. 8. It is first checked if missing data is accidentally described as blank characters (a one-byte space, tab or the like), and the results are reported and corrected (step 800) as described in Function 7-6, using the blank missing report processing section 130. If such a description has occurred, it is displayed on the screen as shown in FIG. 21. It is displayed with emphasis that genotypes are shifted out of place (2100). If the user ticks 2101, the following operation is executed to produce a modified version of the input data. The genotype data 308 for a locus that has caused such a shift is replaced by "0", and each subsequent locus undergoes transcription of the genotype data 308 for its direct preceding locus in the genotype data 308. Also, the relevant data in the input data 309 is replaced by "0".

It is checked if a heterozygous genotype is accidentally described as two alleles separated by a one-byte space, and the results are reported and corrected (step 801) as described in Function 7-8, using the heterozygosis blank report processing section 131. If such a description has occurred, it is displayed on the screen as shown in FIG. 22. It is displayed with emphasis that genotypes are shifted out of place (2200). If the user ticcks 2201, the following operation is executed to produce a modified version of the input data. The genotype data 308 for a locus that has caused such a shift is replaced by a correct heterozygous genotype, and each subsequent locus undergoes transcription of the genotype data 308 for its direct following locus in the genotype data 308. In addition, the last locus (its locus name not specified and having a specified genotype only in the individual having a third or higher-numbered most frequent allele in common) is deleted from the locus data 301 and the genotype data 308. Also, the relevant data in the input data 309 is replaced by the correct heterozygous genotype.

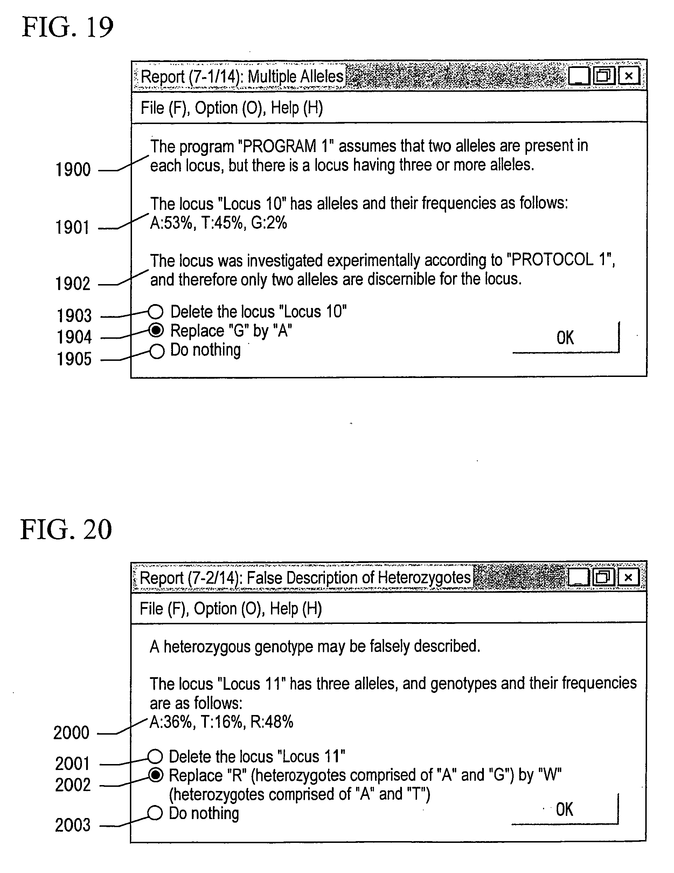

It is checked if a heterozygous genotype is falsely described, and the results are reported and corrected (step 802) as described in Function 7-4, using the falsely described heterozygosis reporting/processing section 129. If there is a locus with a heterozygous genotype falsely described, it is displayed on the screen as shown in FIG. 20. The numeral 2000 in the screen display shows a genotype frequency for the locus summarized from the genotype data 308 for each individual. If the user ticks 2001, the data on the relevant locus is deleted from the locus data 301 and the genotype data 308, and the input data 309 to produce a modified version of the input data. If the user ticks 2002, a correct heterozygous genotype is entered in the genotype data 308 and the input data 309 to produce a modified version of the input data. If the user ticks 2003, nothing is done. Ticks in 2001, 2002 and 2003 are exclusive to each other, and two or more ticks must not be present.

It is checked if a locus having three or more alleles is present, and the results are reported and corrected (step 803) as described in Function 7-1, using the multiple alleles reporting/processing section 128. If the multiple alleles exclusion flag 204 in the program data 133 is TRUE, or the experimental protocol 305 in the input data 134 can discriminate only two alleles, the genotype data 308 in the input data 134 are searched for a locus having three or more alleles. If such a locus is present, it is displayed on the screen as shown in FIG. 19. The numeral 1900 on the display screen is displayed if the multiple alleles exclusion flag 204 in the program data 133 is TRUE. The numeral 1901 shows an allele frequency for the locus summarized from the genotype data 308 for each individual. The numeral 1902 is displayed if the experimental protocol 305 in the input data 134 can discriminate only two alleles. If the user ticks 1903, the data on the relevant locus is deleted from the locus data 301 and the genotype data 308, and the input data 309 to produce a modified version of the input data. If the user ticks 1904, in each individual having a third or higher-numbered most frequent allele, the genotype for the relevant locus in the genotype data 308 and the input data 309 is replaced by a genotype containing the most frequent allele to produce a modified version of the input data. If the user ticks 1905, nothing is done. Ticks in 1903, 1904 and 1905 are exclusive to each other, and two or more ticks must not be present.

Next, it is checked and reported if a blank character is used irregularly (step 804) as described in Function 7-10, using the irregular blank character reporting/processing section 132. In investigating each individual for input data 309, if two or more kinds of blank characters are used as break character for the input data, or two or more blank characters appear in succession, or such characters (a double-byte space or the like) as may be interpreted as either blank character or data are used, blank characters are judged to be used irregularly. If it happens, it is displayed on the screen as shown in FIG. 23. The numeral 2300 expressly shows the types and locations of the blank characters in the input data.

Herein, only the IUB coding system has been described, but the format of data opened by the HapMAP project can also employ the sections used here consisting of: a physical position order report processing section 108; a physical positions overlap report processing section 109; a symbol genotype report processing section 122, a character string genotype report processing section 123, and an unexpected genotype report processing section 124 within a genotype report processing section 111; a multiple alleles report processing section 128 and an irregular blank character report processing section 132 within an allele number report processing section 113; a monomorphism report processing section 114; an in/del report processing section 115; a dual site reaction report processing section 116; a plural populations report processing section 117; a contamination report processing section 118; a special individual report processing section 119; a missing individual report processing section 120; and a reported/corrected items display processing section 121.

Also, the input data format of ARLEQUIN can employ the sections used here consisting of: a symbol genotype report processing section 122 and an unexpected genotype report processing section 124 within a genotype report processing section 111; a falsely described population name report processing section 126 and an unexpected population name report processing section 127 within a population name report processing section 112; a multiple alleles report processing section 128, a blank missing report processing section 130 and an irregular blank character report processing section 132 within an allele number report processing section 113; a monomorphism report processing section 114; an in/del report processing section 115; a dual site reaction report processing section 116; a plural populations report processing section 117; a contamination report processing section 118; a special individual report processing section 119; a missing individual report processing section 120; and a reported/corrected items display processing section 121.

Also, the input data format of LINKAGE can employ the sections used here consisting of: a symbol genotype report processing section 122 and an unexpected genotype report processing section 124 within a genotype report processing section 111; a multiple alleles report processing section 128, a blank missing report processing section 130 and an irregular blank character report processing section 132 within an allele number report processing section 113; a monomorphism report processing section 114; an in/del report processing section 115; a dual site reaction report processing section 116; a plural populations report processing section 117; a contamination report processing section 118; a special individual report processing section 119; a missing individual report processing section 120; and a reported/corrected items display processing section 121.

Herein, each type of error has been described using an error made at a single locus in a single individual, but can be also described in the same manner using errors made at plural loci in plural individuals. Specifically, as an example, only a single individual (P07) having many missing data is described in FIG. 30, but plural individuals may actually have many missing data. Such a case can be dealt with similarly. Specifically, every individual having many missing data can be listed up on the illustrative display screen shown in FIG. 30. It applies to other types of error similarly.

Herein, the whole sample population has been checked in a lump using the monomorphism report processing section 114 or the plural populations report processing section 117, but each population may be checked differently instead. Specifically, using the monomorphism report processing section 114, for example, it may be checked as such a case if there is a locus which may be polymorphic in the healthy population, but is not polymorphic in the patient population.

The data input support system for gene analysis according to the present invention has been described hereinbefore by means of specific embodiments, but the present invention is not limited thereto. Those skilled in the art could make various alterations or modifications in the constitutions and functions of the invention which may be associated with the foregoing or other embodiments, within the gist of the present invention.

The data input support system for gene analysis according to the present invention is available on a computer comprising memory means, input means, display means and the like, wherein information processing consisting of detection and display of certain types of errors in the input data of genotypes can be actually achieved by use of hardware resources such as memory means, input means and display means described above. Accordingly, the system applies to a technical idea utilizing natural laws, and can be industrially utilized in medical and/or biological research institutions and the likes which are engaged in linkage disequilibrium analysis.

## Claims

1. A data input support system for inspecting genotype data input into a program for linkage disequilibrium analysis, wherein the system comprises:
a storage section for retaining error types for genotype data corresponding to the program for linkage disequilibrium analysis;
an error detection section for checking the input genotype data for the error types and detecting errors; and
an error report/display section for displaying the report of the detected errors.

2. The data input support system according to claim 1, further comprising error correction means which accepts an input for correcting the reported error in the input genotype data and corrects the genotype data based on the input.

3. The data input support system according to claim 2, wherein the error correction means accepts a correction input by which for the locus having three or more alleles, a third or higher-numbered most frequent allele of the three or more alleles is rewritten into a first or higher-numbered most frequent allele, and thereby corrects the genotype data in such a manner.

4. The data input support system according to any one of claims 1 to 3, further comprising means for displaying as a list the content of errors reported by the error report/display section as well as the content of corrections for the genotype data made by the error correction means.
